# EUROPEAN PATENT SPECIFICATION

(11) **EP 3 995 008 B1**
(45) Date of publication and mention of the grant of the patent: **31.05.2023**
(21) Application number: 20206007.5
(22) Date of filing: 05.11.2020
(51) Int. Cl.: A24F 40/65, H04L 67/12, H04W 4/021, G06Q 50/10, G01S 19/42, H04W 4/38, G06Q 30/02, H04W 4/02, A61M 15/06, A24F 40/53, H04L 67/52

(54) **AEROSOL GENERATING DEVICE FOR VAPING INCLUDING AN ENVIRONMENT SENSOR, AND SYSTEM FOR PROVIDING INFORMATION TO USERS ABOUT PLACES TO VAPE**
AEROSOLERZEUGUNGSVORRICHTUNG ZUM VAPEN MIT EINEM UMGEBUNGSSENSOR UND SYSTEM ZUR BEREITSTELLUNG VON INFORMATIONEN FÜR BENUTZER ÜBER PLÄTZE ZUM VAPEN
DISPOSITIF DE GÉNÉRATION D'AÉROSOL POUR VAPOTAGE COMPRENANT UN CAPTEUR D'ENVIRONNEMENT ET SYSTÈME PERMETTANT DE FOURNIR DES INFORMATIONS SUR LES LIEUX DE VAPOTAGE AUX UTILISATEURS

(43) Date of publication of application: 11.05.2022
(73) Proprietor: JT International S.A., 1202 Geneva (CH)
(72) Inventor: YAMAGUCHI, Akira, 1207 GENEVA (CH)
(74) Representative: Santarelli

(56) References cited:
- WO-A1-2017/205692
- WO-A2-2014/205456
- US-A1- 2018 177 231
- US-B2- 9 155 337
- BO LI ET AL: "i-Function of Electronic Cigarette: Building Social Network by Electronic Cigarette", 2011 INTERNATIONAL CONFERENCE ON INTERNET OF THINGS AND 4TH INTERNATIONAL CONFERENCE ON CYBER, PHYSICAL AND SOCIAL COMPUTING, 19 October 2011 (2011-10-19), pages 634-637, XP055535276, DOI: 10.1109/iThings/CPSCom.2011.124 ISBN: 978-0-7695-4580-6

## Description

### Field of the invention

The present invention relates to aerosol generating devices for vaping, such as electronic cigarettes (also named e-cigarettes) and heated though not burnt tobacco devices.

An electronic cigarette generally comprises an atomizer or clearomizer, a power source such as a battery, and a container such as a cartridge or a tank receiving an e-liquid to be atomized by heating (by means of an electrically controlled heater) in the atomizer or clearomizer.

A heated though not burnt tobacco device generally comprises a pen-like holder, in which a tobacco stick is inserted, which is then heated up to 250°C to 350°C by means of an electrically controlled heater.

A tobacco stick is a stick which looks like a traditional cigarette and includes a reconstituted tobacco material, a filter which cools the aerosol and a soft mouthpiece which mimics the feel of a traditional cigarette. The reconstituted tobacco material may be made of a dried tobacco suspension including tobacco, humectants (like water and glycerine) to encourage aerosol formation, binding agents and aroma agents.

Aerosol generating devices for vaping are thus portable devices comprising an electric heat source that heats a vaporizable product (e-liquid or reconstituted tobacco material or other vaporizable material) to create an aerosol that the user inhales.

### Background of the invention

Smoking traditional cigarettes is considered as being disturbing and even dangerous for the persons attending around the smoker. Smoking is thus forbidden in lots of places.

On the contrary, vaping is often allowed or tolerated where smoking is forbidden. Nevertheless, a vaper (user of an aerosol generating device for vaping) may be afraid of disturbing the persons around him even in a place where vaping is allowed, and he thus may feel uncomfortable to generate a big cloud. On the other hand, if there is nobody, he should be able to enjoy a big cloud.

Therefore, in order to fully enjoy a vaping moment, it could be appreciated for the vaper to be informed of the good places to vape nearby or in a given area, a good place to vape being a place where the vaper could vape freely and to enjoy big clouds without the risk of disturbing other people.

US9155337 discloses an electronic cigarette comprising a housing, an atomizer and a control circuit. The e-cigarette further comprises a camera configured to capture images of nearby persons. The captured images are used to determine specific persons, or persons of specified demographics (e.g. children) being nearby, and the control circuit is configured to limit (e.g. prevent, reduce, or modify) the use of the e-cigarette based on the presence of a proximate person or on the identity or the nature (e.g. children) of the persons nearby.

The device disclosed by US9155337 cannot provide the user with information about the places to vape in a given area.

The present invention aims to solve this problem.

### Summary of the invention

The present invention thus relates to an aerosol generating device for vaping, comprising:
- an atomizer for generating a vapor,
- a microcontroller for controlling the atomizer,
- a positioning sensor for detecting the location of the aerosol generating device for vaping, the positioning sensor being connected with the microcontroller,
- a communication interface for sending data to a remote server (here after simply referred to as "a server" or "the server").

The aerosol generating device for vaping according to the invention is characterized in that:
- it further comprises an environment sensor, connected to the microcontroller, able to detect any person situated in a given radius there around and to estimate the distance between the environment sensor and each detected person,
- the microcontroller is configured to calculate the number of persons detected by the environment sensor and to determine a minimum distance between the environment sensor and the detected persons,
- the microcontroller is configured to send to the server the detected location of the aerosol generating device for vaping together with a number of environmental data including the calculated number of detected persons and the determined minimum distance.

The invention also relates to a system and a method for providing information to users about places to vape, which uses a number of aerosol generating devices as defined above. These system and method will be described here later.

The microcontroller may be configured to send the environmental data with the detected location in real time or to collect this information and send them in batch upon request by the user or on a regular period basis for example every minutes.

Advantageously, the environment sensor is a LIDAR. In a preferred embodiment, the environment sensor is a 360° LIDAR.

According to a possible feature of the invention, the communication interface of the aerosol generating device for vaping includes a WIFI emitter.

According to a possible feature of the invention, the microcontroller is moreover configured to determine the amount of vapor generated and to adjust the amount of vapor generated by varying one or more operating parameters of the atomizer on the basis of the calculated number of detected persons and the determined minimum distance. Thus, the amount of vapor is always and automatically adjusted depending on the situation (crowded or unfilled place), to provide comfortable and optimal delivery in each situation.

The atomizer of the aerosol generating device for vaping may include a heater for heating a vaporizable substance to generate the vapor. Note that the vaporizable substance may be a solid vaporizable material or an e-liquid. According to a possible feature of the invention, the operating parameters of the atomizer, which may be controlled by the microcontroller to adjust the amount of vapor generated, are chosen among the temperature of the heater (this temperature may be detected by a temperature sensor or calculated by the microcontroller according to other parameters), a current or voltage supplied to the heater, an opening of an air flow inlet or passage, a heated amount of the vaporizable substance being heated by the heater.

As mentioned above, in a second aspect, the invention also relates to a system for providing information to users about places to vape. This system comprises:
- a number of aerosol generating devices for vaping according to the invention,
- a sever, configured to receive the detected location and the environmental data from each of the number of aerosol generating devices for vaping, the server including a classifying module for evaluating a level of comfort for each received location depending on the associated environmental data, and a mapping module for generating a list of places to vape listing all the received locations together with the corresponding evaluated level of comfort.

Advantageously, the list generated by the mapping module takes the form of a geographic map where the locations received from each of the number of aerosol generating devices for vaping are marked and where each marked location is matched with a sign, for example a color code, representative of the level of comfort evaluated for said location.

As mentioned above, in a third aspect, the invention also relates to a method for providing information to users about places to vape. This method comprises the following steps:
- using a system as previously described,
- from each of the number of aerosol generating devices for vaping, reception by the server of the detected location of the aerosol generating device for vaping together with the environmental data,
- evaluation by the server (more precisely by a classifying module thereof) of a level of comfort for each received location, depending on the environmental data received together with said location,
- generation by the server (more precisely by a mapping module thereof) of a list of places to vape listing all the received locations together with the evaluated level of comfort,
- sending this list to a user's smartphone.

Therefore, in a fourth aspect, the invention also relates to an application for smartphone which, when loaded and run on the smartphone, causes the reception and the displaying of the list of places to vape generated in the method according to the invention. The list of places to vape may be received upon request by the user via the application or may be received and updated in real time as soon and as long as the application is running.

The list of places to vape preferably takes the form of a geographic map of the area around the current location of the user as detected either by the positioning sensor of the user's aerosol generating device for vaping (a user ID being associated with the user's vaping device and being previously entered in the application) or, preferably, by the GPS of the smartphone where the application is running.

This geographic map may be centered on the current location of the smartphone and may be updated in real time or as soon as a change in location of the smartphone is detected, such as to follow the travel of the smartphone (and the user).

According to a possible feature of the invention, the aerosol generating device for vaping includes a control tool for deactivating the environment sensor, whereby allowing the user to prevent the scanning of the environment by the environment sensor or to prevent the use by the microcontroller of the data collected by the environment sensor. This control tool for deactivating the environment sensor may be a button or touch command for interrupting the connection between the sensor and the microcontroller or a human to machine interface (such as a digital touch screen) allowing the user to enter a request to prevent the sending by the microcontroller to the server of the detected environmental data and location of the aerosol generating device for vaping.

### Brief description of the drawings

Other particularities and advantages of the invention will also emerge from the following description.

In the accompanying drawings, given by way of non-limiting examples:
- Figure 1 represents, in a three dimensional view, a heated though not burnt tobacco device according to a first embodiment of the invention;
- Figure 2 represents, in a very schematic longitudinal cross section view, the heated though not burnt tobacco device illustrated at Figure 1;
- Figure 3 represents, in a side view, an e-cigarette according to a second embodiment of the invention;
- Figure 4 represents, in a very schematic longitudinal cross section view, a first part of the e-cigarette illustrated at Figure 3;
- Figure 5 represents, in a schematic exploded view, the second part of the e-cigarette illustrated at Figure 3;
- Figure 6 represents, in a schematic three dimensional view, a system according to the invention for providing information to users about places to vape.

### Detailed Description

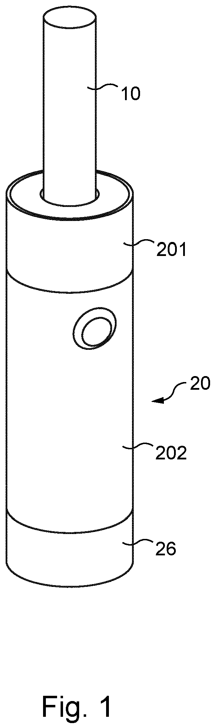

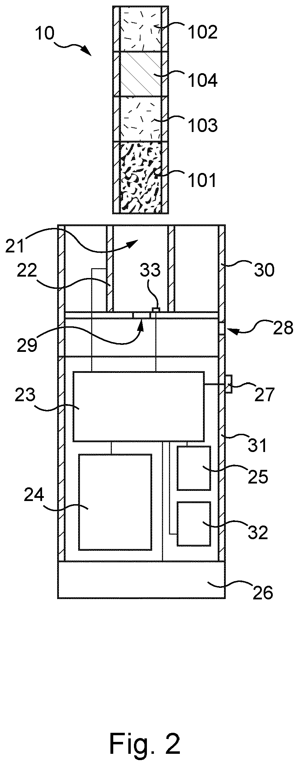

Fig. 1 and 2 show a heated though not burnt tobacco device according to the invention, which comprises a pen-like holder 20 adapted to receive a tobacco stick 10.

In a conventional way, the tobacco stick 10 includes a vaporizable material 101 at a lower part and a mouthpiece 102 at an upper part. It may further comprise a filter section 103 and a cooler section 104 between the vaporizable material 101 and the mouthpiece 102.

The pen-like holder 20 comprises a vaporizing unit 201, referred to as an atomizer 201 where the vaporizable material embedded in the tobacco stick 10 can be vaporized, and a powering unit 202 which comprises a housing 31 accommodating a microcontroller 23 and a power source such as a battery 25. The microcontroller 23 may consist in a main printed circuit board assembly. It is connected to the battery 25 and powered by the latter in a conventional way (schematically represented by a connecting line). The bottom end of the powering unit 202 may include connecting means (not shown) for connecting the battery 25 to a charger (not shown) supplied with electricity by a suitable transformer or by a USB (Universal Serial Bus) socket.

The atomizer 201 comprises a housing 30 which accommodates a heating cavity 21 configured to receive the lower part of the tobacco stick 10. This heating cavity 21 is surrounded by a heater 22 which is operated by the microcontroller 23. The vaporizable material 101 placed in the heating cavity 21 can deliver fine droplets when heated by the heater 22.

At least one air inlet 28 is provided in the atomizer housing 30. An air passage 29 is also provided in the bottom wall of the heating cavity. Therefore, when a user puffs on the mouthpiece 102 of the tobacco stick, an airflow is created through the air inlet 28 and the air passage 29 then through the stick. The fine droplets generated in the vaporizable material 101 heated by the heater 22 are carried out in this airflow, forming a vapor inhaled by the user.

The atomizer may include a motorized airflow regulating shroud or a motorized airflow regulating flange (not shown) operated by the microcontroller, the rotation of which modifies respectively the opening of the air inlet 28 or the opening of the air passage 29 in order to regulate the amount of vapor generated by the atomizer. Another way to regulate the amount of vapor generated by the atomizer is to adjust the current or voltage supplied to the heater 22. Another way to regulate the amount of vapor generated by the atomizer is to provide the atomizer with a segmented heater including several sections along the axis of the stick and to selectively operate only one section or the entirety of the heater to partly or entirely heat the vaporizable material, whereby regulating the amount of droplets generated in the vaporizable material.

The atomizer may further comprise a temperature sensor 33 for detecting the temperature in the heating cavity 21, and the microcontroller 23 may be configured to adjust the current or voltage supplied to the heater or the opening of the air inlet or the air passage depending on the temperature in the cavity, for the purpose of regulating the amount of vapor generated.

Whatever the operating parameter (airflow, current or voltage supplied to the heater, etc.) controlled by the microcontroller 23, the amount of vapor generated is advantageously regulated depending on the environmental situation, as explained after.

The powering unit 202 may further comprise a ON/OFF switch 27 connected to the microcontroller for causing the microcontroller to allow or stop power supplying the heater 22. In an alternative embodiment, the atomizer includes a pressure sensor and the microcontroller is configured to cause the heater 22 to be supplied by the battery 24 when a drop in pressure corresponding to a puff by the user is detected.

According to the invention, the powering unit 202 of the pen-like holder further comprises: an environment sensor such as a 360°LIDAR 26 for scanning the environment of the heated though not burnt tobacco device; a positioning sensor such as a GPS sensor 32 for detecting the location of the heated though not burnt tobacco device; a communication interface such as a WIFI emitter 25 for sending data to a remote server. The LIDAR 26, the GPS sensor 32, and the WIFI emitter 25 are all connected to the microcontroller 23.

In another embodiment, a heated though not burnt tobacco device according to the invention may be a device configured to receive pods or capsules or tablets of vaporizable material instead of tobacco sticks. The vaporizable material compacted in tablets or embedded in capsules or pods may even not comprise natural tobacco. In another embodiment, the heated though not burnt tobacco device according to the invention may be configured to receive loose-leaf tobacco.

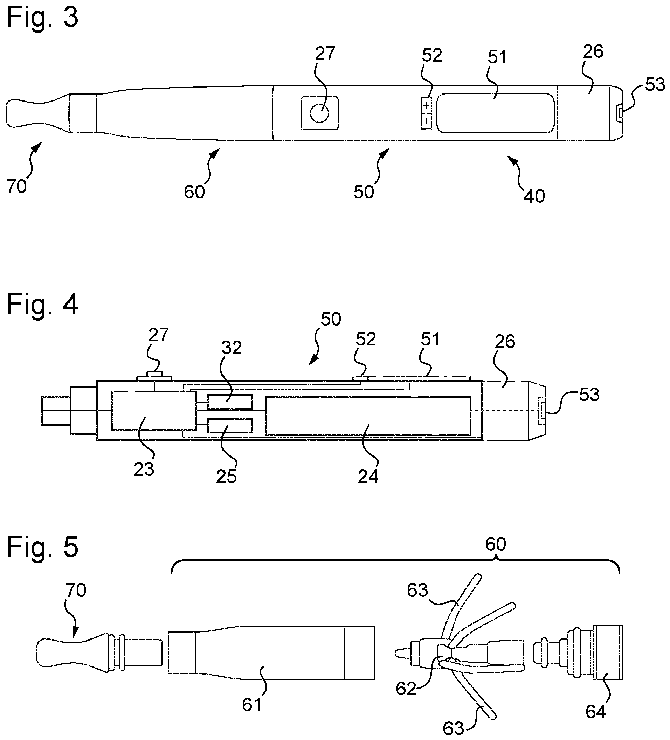

The invention also applies to e-cigarettes, such as the e-cigarette 40 illustrated at Fig. 3 and 4. The e-cigarette 40 comprises a powering unit 50, a vaporizing unit 60, here referred to as an "atomizer" although sometimes known as a "clearomizer", and a drip tip forming a mouthpiece 70.

In a conventional way, the atomizer 60 comprises a tank 61 for receiving an e-liquid to be vaporized, a heater consisting in a heating resistance coil 62 through which a plurality of wicks 62 are passing, and a threaded base 64 for connecting the atomizer 60 to the powering unit 50.

As in the above described heated though not burnt tobacco device, the powering unit 50 of the e-cigarette accommodates a microcontroller 23, a power source 24 such as a battery, a positioning sensor 32, a communication interface 25 for communicating data to a remote server and an environment sensor 26 such as a LIDAR, as well as a ON/OFF switch 27. The powering unit may further comprise a LED digital display 51 for displaying operating information such as volts, watts, ohms and puffs, and +/- power and voltage buttons 52 for regulation by the user of the current or voltage supplied to the heating resistance coil 62, and a micro USB socket 53 for charging the power source 24.

According to the invention, both for the heated though not burnt tobacco device of Fig. 1 and 2 and for the e-cigarette of Fig. 3 and 4, the LIDAR (or other environment sensor) 26 and the microcontroller 23 are configured to detect any person situated in a given radius around the LIDAR and to estimate the distance between the LIDAR and each detected person, then to calculate the number of persons detected by the LIDAR and to determine a minimum distance between the LIDAR and the detected persons.

The microcontroller 23 is further configured to associate the calculated number of persons and the determined minimum distance (referred to as "environmental data") with location data received from the GPS sensor 32, and to send all these data to a remote server thanks to the WIFI emitter 25.

Besides, the microcontroller 23 is preferably also configured to regulate the amount of vapor generated by the heater depending on at least one environmental data such as the calculated number of persons and/or the determined minimum distance, in order to automatically adapt the vapor delivery to the environmental situation, whereby the user is sure to not disturb the persons nearby without the need of thinking of adapting himself his way to vape.

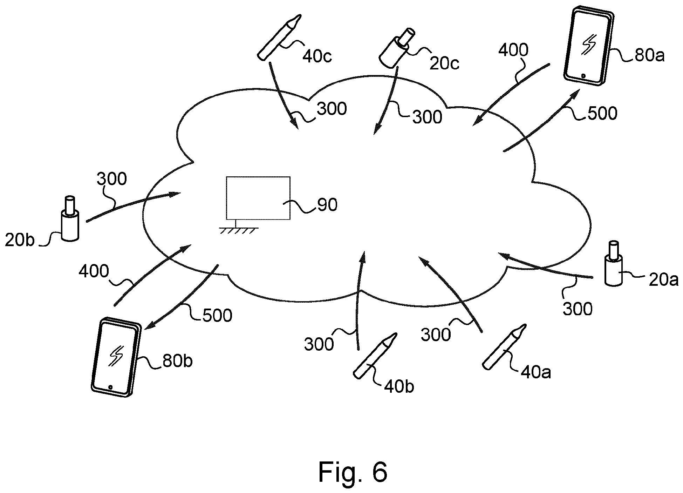

In another aspect, the invention proposes a system for providing information to users about places to vape as illustrated at Fig. 6. This system includes:
- a server 90 connected to the cloud,
- a number of aerosol generating devices for vaping such as heated though not burnt tobacco devices 20a, 20b, 20c and e-cigarettes 40a, 40b, 40c, here after simply referred to as "vaping devices", all equipped with a positioning sensor able to detect the location of the device, an environment sensor able to detect the persons around and a communication interface able to transmit data to the server 90 via the cloud,
- at least one user's smartphone 80a, 80b able to communicate with the server 90 via the cloud.

In another aspect, the invention proposes a method for providing information to users about places to vape using the above described system and wherein:
- at each vaping device 20a, 20b, 20c, 40a, 40b, 40c of the system, the environment sensor 26 (LIDAR) of the vaping device scans the environment around the vaping device, such as to detect any person nearby and to estimate the distance between the vaping device and each detected person; this scan may be performed as soon as the vaping device is switched on but it also may be prevented by the user as explained later; the microcontroller 23 of the vaping device then calculates the number of persons detected by the environment sensor 26 and determines a minimum distance between the vaping device and the detected persons; next the microcontroller 23 prepares a set of data to be sent to the server 90, including the location detected by the positioning sensor (GPS) 32 of the vaping device and the number of persons previously calculated and the minimum distance previously determined; this cycle is repeated on a regular period basis, for example every minute.
- as soon as a set of data to be sent (environmental data + location) is thus prepared in a vaping device of the system, the microcontroller of this vaping device sends to the server 90 (arrow 300) the prepared set of data via the cloud, using the communication interface 25 of the vaping device,
- the server 90 thus receives from all the vaping devices of the system, a lot of sets of data including the locations of the vaping devices and the environmental data detected by each of them;
- for each set of data received, the server 90 evaluates a level of comfort for the corresponding location, depending on the environmental data of the set;
- the server 90 establishes a list of locations with their corresponding level of comfort as previously evaluated; this list is updated in real time. It may take the form of a geographic map of places to vape with color codes representing the levels of comfort of the listed locations;
On the other hand:
- a user who wants to get information about places to vape can use his smartphone 80a, 80b where an application can be downloaded to this end;
- when the application is started, a request for information (arrow 400), is sent to the server 90 via the cloud, together with the location of the user detected by the GPS of the smartphone;
- upon receipt of this request, the server 90 sends to the smartphone (arrow 500) a part of the map of places to vape corresponding to the area around the location of the smartphone.

To be noted that a button for deactivating the LIDAR of a vaping device, for example by interrupting the connection between the LIDAR and the microcontroller, may be provided to allow the user not to collect or not to send environmental data when he wants, for example when he is at home or in a private place which is not intended to be considered as a place to vape by the other users of the system.

The invention is not limited to the illustrated embodiments. In particular, the invention applies to any vaping device, including vaping devices using pods or capsules or loose-leaf tobacco or vaping devices having heaters structurally different from the heaters illustrated in the annexed drawings. Besides, as another example of alternative embodiments according to the invention, the environment sensor of the vaping device may not be a LIDAR, as soon as this sensor is able to detect any person nearby and to evaluate the distance between the detected person and the sensor.

## Claims

1. An aerosol generating device for vaping (20, 40), comprising:
- an atomizer (201, 60) for generating a vapor,
- a microcontroller (23) for controlling the atomizer,
- a positioning sensor (32) for detecting the location of the aerosol generating device for vaping, the positioning sensor being connected with the microcontroller,
- a communication interface (25) for sending data to a server (90),
**characterized in that**:
• the aerosol generating device for vaping further comprises an environment sensor (26), connected to the microcontroller (23), able to detect any person situated in a given radius there around and to estimate the distance between the environment sensor (26) and each detected person,
• the microcontroller (23) is configured to calculate the number of persons detected by the environment sensor (26) and to determine a minimum distance between the environment sensor and the detected persons,
• the microcontroller (23) is configured to send to the server, by means of the communication interface (25), the detected location of the aerosol generating device for vaping together with a number of environmental data including the calculated number of detected persons and the determined minimum distance.

2. The aerosol generating device for vaping according to claim 1, wherein the environment sensor (26) is a LIDAR.

3. The aerosol generating device for vaping according to claim 1, wherein the environment sensor (26) is a 360° LIDAR.

4. The aerosol generating device for vaping according to any one of claims 1 to 3, wherein the communication interface (25) includes a WIFI emitter.

5. The aerosol generating device for vaping according to any one of claims 1 to 4, wherein the microcontroller (23) is configured to determine the amount of vapor generated and to adjust the amount of vapor generated by varying one or more operating parameters of the atomizer (201, 60), on the basis of the calculated number of detected persons and the determined minimum distance.

6. The aerosol generating device for vaping according to claim 5, wherein the atomizer includes a heater (22, 62) for heating a vaporizable substance to generate the vapor, and the operating parameters of the atomizer are chosen among the temperature of the heater, a current or voltage supplied to the heater, an opening of an air flow inlet or passage (28, 29), a heated amount of the vaporizable substance being heated by the heater.

7. The aerosol generating device for vaping according to any one of claims 1 to 6, including a control tool for deactivating the environment sensor (26).

8. A system for providing information to users about places to vape, comprising:
• a number of aerosol generating devices for vaping (20a, 20b, 20c, 40a, 40b, 40c) according to any one of claims 1 to 7,
• a sever (90), configured to receive the detected location and the environmental data from each of the number of aerosol generating devices for vaping, the server including a classifying module for evaluating a level of comfort for each location depending on the environmental data received and a mapping module for generating a list of places to vape listing all the received locations together with the evaluated level of comfort.

9. The system for providing information to users about places to vape according to claim 8, wherein the list has the form of a geographic map where the locations received from each of the number of aerosol generating devices for vaping (20a, 20b, 20c, 40a, 40b, 40c) are marked and where each marked location is matched with a sign representative of the level of comfort evaluated for said location.

10. A method for providing information to users about places to vape, comprising:
• using a system according to claim 8 or 9,
• from each of the number of aerosol generating devices for vaping (20a, 20b, 20c, 40a, 40b, 40c), reception by the server (90) of the detected location of the aerosol generating device for vaping together with the environmental data,
• evaluation by the server (90) of a level of comfort for each received location, depending on the environmental data received together with said location,
• generation by the server (90) of the list of places to vape listing all the received locations together with the evaluated level of comfort.

11. The method according to claim 10, further comprising: sending this list to a user's smartphone (80a, 80b).

12. An application for smartphone which, when loaded and run on the smartphone, causes the reception and the displaying of the list of places to vape generated in the method according to claim 10 or 11.

## Patentansprüche

1. Aerosolerzeugungsvorrichtung zum Vapen (20, 40), umfassend:
- einen Zerstäuber (201, 60) zum Erzeugen von Dampf,
- eine Mikrosteuerung (23) zum Steuern des Zerstäubers,
- einen Positionierungssensor (32) zum Erkennen der Stelle der Aerosolerzeugungsvorrichtung zum Vapen, wobei der Positionierungssensor mit der Mikrosteuerung verbunden ist,
- eine Kommunikationsschnittstelle (25) zum Senden von Daten an einen Server (90),
**dadurch gekennzeichnet, dass**:
• die Aerosolerzeugungsvorrichtung zum Vapen weiter einen Umgebungssensor (26) umfasst, der mit der Mikrosteuerung (23) verbunden ist, imstande ist, jede Person zu erkennen, die sich in einem gegebenen Radius dort rund um befindet, und die Distanz zwischen dem Umgebungssensor (26) und jeder erkannten Person zu schätzen,
• die Mikrosteuerung (23) konfiguriert ist, um die Anzahl an Personen zu berechnen, die durch den Umgebungssensor (26) erkannt wird, und eine Mindestdistanz zwischen dem Umgebungssensor und den erkannten Personen zu bestimmen,
• die Mikrosteuerung (23) konfiguriert ist, um die erkannte Stelle der Aerosolerzeugungsvorrichtung zum Vapen gemeinsam mit einer Anzahl an Umgebungsdaten, die die berechnete Anzahl an erkannten Personen und die bestimmte Mindestdistanz beinhalten, anhand der Kommunikationsschnittstelle (25) an den Server zu senden.

2. Aerosolerzeugungsvorrichtung zum Vapen nach Anspruch 1, wobei der Umgebungssensor (26) ein LIDAR-Sensor ist.

3. Aerosolerzeugungsvorrichtung zum Vapen nach Anspruch 1, wobei der Umgebungssensor (26) ein 360° LIDAR-Sensor ist.

4. Aerosolerzeugungsvorrichtung zum Vapen nach einem der Ansprüche 1 bis 3, wobei die Kommunikationsschnittstelle (25) einen WLAN-Sender beinhaltet.

5. Aerosolerzeugungsvorrichtung zum Vapen nach einem der Ansprüche 1 bis 4, wobei die Mikrosteuerung (23) konfiguriert ist, um die erzeugte Dampfmenge zu bestimmen, und die erzeugte Dampfmenge durch Ändern eines oder mehrerer Betriebsparameter des Zerstäubers (201, 60) auf der Grundlage der berechneten Anzahl an erkannten Personen und der Mindestdistanz anzupassen.

6. Aerosolerzeugungsvorrichtung zum Vapen nach Anspruch 5, wobei der Zerstäuber einen Erhitzer (22, 62) zum Erhitzen einer verdampfbaren Substanz zum Erzeugen des Dampfes beinhaltet, und die Betriebsparameter des Zerstäubers aus der Temperatur des Erhitzers, dem Strom oder der Spannung, der/die dem Erhitzer zugeführt wird, einer Öffnung eines Luftstromeinlasses oder -durchlasses (28, 29), einer erhitzten Menge der verdampfbaren Substanz, die durch den Erhitzer erhitzt wird, ausgewählt werden.

7. Aerosolerzeugungsvorrichtung zum Vapen nach einem der Ansprüche 1 bis 6, die ein Steuerungsinstrument zum Deaktivieren des Umgebungssensors (26) beinhaltet.

8. System zum Bereitstellen von Informationen für Nutzer über Plätze zum Vapen, umfassend:
• eine Anzahl an Aerosolerzeugungsvorrichtungen zum Vapen (20a, 20b, 20c, 40a, 40b, 40c) nach einem der Ansprüche 1 bis 7,
• einen Server (90), der konfiguriert ist, um die erkannte Stelle und die Umgebungsdaten aus jeder der Anzahl an Aerosolerzeugungsvorrichtungen zum Vapen zu empfangen, wobei der Server ein Klassifizierungsmodul zum Bewerten einer Komfortstufe für jede Stelle in Abhängigkeit von den empfangenen Umgebungsdaten, und ein Zuweisungsmodul zum Erzeugen einer Liste an Plätzen zum Vapen beinhaltet, die alle empfangenen Stellen gemeinsam mit der bewerteten Komfortstufe auflistet.

9. System zum Bereitstellen von Informationen für Nutzer über Plätze zum Vapen nach Anspruch 8, wobei die Liste die Form einer geografischen Karte aufweist, auf der die Stellen, die von jeder der Anzahl an Aerosolerzeugungsvorrichtungen zum Vapen (20a, 20b, 20c, 40a, 40b, 40c) empfangen werden, vermerkt sind, und auf der jede vermerkte Stelle mit einem Zeichen übereinstimmt, das repräsentativ für die Komfortstufe ist, die für die Stelle bewertet wurde.

10. Verfahren zum Bereitstellen von Informationen für Nutzer über Plätze zum Vapen, umfassend:
• Verwenden eines Systems nach Anspruch 8 oder 9,
• von jeder der Anzahl an Aerosolerzeugungsvorrichtungen zum Vapen (20a, 20b, 20c, 40a, 40b, 40c) Empfangen durch den Server (90) der erkannten Stelle der Aerosolerzeugungsvorrichtung zum Vapen gemeinsam mit den Umgebungsdaten,
• Bewerten durch den Server (90) einer Komfortstufe für jede empfangene Stelle, in Abhängigkeit von den Umgebungsdaten, die gemeinsam mit der Stelle empfangen werden,
• Erzeugen durch den Server (90) der Liste an Plätzen zum Vapen, die alle empfangenen Stellen gemeinsam mit der bewerteten Komfortstufe auflistet.

11. Verfahren nach Anspruch 10, weiter umfassend: Senden dieser Liste an ein Smartphone (80a, 80b) eines Nutzers.

12. App für ein Smartphone, die, wenn sie auf das Smartphone geladen wird und läuft, den Empfang und die Anzeige der Liste an Plätzen zum Vapen bewirkt, die in dem Verfahren nach Anspruch 10 oder 11 erzeugt wird.

## Revendications

1. Dispositif de génération d'aérosol pour vapotage (20, 40), comprenant :
- un atomiseur (201, 60) pour générer une vapeur,
- un microdispositif de commande (23) pour commander l'atomiseur,
- un capteur de positionnement (32) pour détecter l'emplacement du dispositif de génération d'aérosol pour vapotage, le capteur de positionnement étant connecté au microdispositif de commande,
- une interface de communication (25) pour envoyer des données à un serveur (90), **caractérisé en ce que** :
• le dispositif de génération d'aérosol pour vapotage comprend en outre un capteur d'environnement (26), connecté au microdispositif de commande (23), capable de détecter toute personne située dans un rayon donné autour de lui et d'estimer la distance entre le capteur d'environnement (26) et chaque personne détectée,
• le microdispositif de commande (23) est configuré pour calculer le nombre de personnes détectées par le capteur d'environnement (26) et déterminer une distance minimale entre le capteur d'environnement et les personnes détectées,
• le microdispositif de commande (23) est configuré pour envoyer au serveur, au moyen de l'interface de communication (25), l'emplacement détecté du dispositif de génération d'aérosol pour vapotage, conjointement avec un nombre de données environnementales comportant le nombre de personnes détectées calculé et la distance minimale déterminée.

2. Dispositif de génération d'aérosol pour vapotage selon la revendication 1, dans lequel le capteur d'environnement (26) est un LIDAR.

3. Dispositif de génération d'aérosol pour vapotage selon la revendication 1, dans lequel le capteur d'environnement (26) est un LIDAR 360 °.

4. Dispositif de génération d'aérosol pour vapotage selon l'une quelconque des revendications 1 à 3, dans lequel l'interface de communication (25) comporte un émetteur WIFI.

5. Dispositif de génération d'aérosol pour vapotage selon l'une quelconque des revendications 1 à 4, dans lequel le microdispositif de commande (23) est configuré pour déterminer la quantité de vapeur générée et pour régler la quantité de vapeur générée en faisant varier un ou plusieurs paramètres de fonctionnement de l'atomiseur (201, 60), sur la base du nombre de personnes détectées calculé et de la distance minimale déterminée.

6. Dispositif de génération d'aérosol pour vapotage selon la revendication 5, dans lequel l'atomiseur comporte un dispositif de chauffage (22, 62) pour chauffer une substance vaporisable pour générer de la vapeur, et les paramètres de fonctionnement de l'atomiseur sont choisis parmi la température du dispositif de chauffage, un courant ou une tension fourni(e) au dispositif de chauffage, une ouverture d'une entrée ou d'un passage de flux d'air (28, 29), une quantité chauffée de la substance vaporisable étant chauffée par le dispositif de chauffage.

7. Dispositif de génération d'aérosol pour vapotage selon l'une quelconque des revendications 1 à 6, comportant un outil de commande pour désactiver le capteur d'environnement (26).

8. Système pour fournir des informations à des utilisateurs sur des lieux pour vapoter, comprenant :
• un nombre de dispositifs de génération d'aérosol pour vapotage (20a, 20b, 20c, 40a, 40b, 40c) selon l'une quelconque des revendications 1 à 7,
• un serveur (90), configuré pour recevoir l'emplacement détecté et les données environnementales provenant de chacun du nombre de dispositifs de génération d'aérosol pour vapotage, le serveur comportant un module de classification pour évaluer un niveau de confort pour chaque emplacement en fonction des données environnementales reçues et un module de cartographie pour générer une liste de lieux pour vapoter listant tous les emplacements reçus conjointement avec le niveau de confort évalué.

9. Système pour fournir des informations à des utilisateurs sur des lieux pour vapoter selon la revendication 8, dans lequel la liste présente la forme d'une carte géographique où les emplacements reçus de chacun du nombre de dispositifs de génération d'aérosol pour vapotage (20a, 20b, 20c 40a, 40b, 40c) sont marqués et où chaque emplacement marqué est associé à un signe représentatif du niveau de confort évalué pour ledit emplacement.

10. Procédé pour fournir des informations à des utilisateurs sur des lieux pour vapoter, comprenant :
• l'utilisation d'un système selon la revendication 8 ou 9,
• depuis chacun du nombre de dispositifs de génération d'aérosol pour vapotage (20a, 20b, 20c, 40a, 40b, 40c), la réception par le serveur (90) de l'emplacement détecté du dispositif de génération d'aérosol pour vapotage conjointement avec les données environnementales,
• l'évaluation par le serveur (90) d'un niveau de confort pour chacun des emplacements reçus, en fonction des données environnementales reçues conjointement avec ledit emplacement,
• la génération par le serveur (90) de la liste de lieux pour vapoter listant tous les emplacements reçus conjointement avec le niveau de confort évalué.

11. Procédé selon la revendication 10, comprenant en outre : l'envoi de cette liste au téléphone intelligent d'un utilisateur (80a, 80b).

12. Application pour téléphone intelligent qui, lorsqu'elle est chargée et exécutée sur le téléphone intelligent, entraîne la réception et l'affichage de la liste de lieux pour vapoter générée par le procédé selon la revendication 10 ou 11.
